# EUROPEAN PATENT SPECIFICATION

(11) **EP 2 398 517 B1**
(45) Date of publication and mention of the grant of the patent: **20.04.2016**
(21) Application number: 10743393.0
(22) Date of filing: 18.02.2010
(51) Int. Cl.: A61L 27/06, A61L 27/22, A61L 27/34, A61L 27/54

(54) **ANTIBACTERIAL SURFACE AND METHOD OF FABRICATION**
ANTIBAKTERIELLE OBERFLÄCHE UND VERFAHREN ZU IHRER HERSTELLUNG
SURFACE ANTIBACTÉRIENNE ET SON PROCÉDÉ D'OBTENTION

(30) Priority: 19.02.2009 US 153840 P; 20.02.2009 US 154278 P
(43) Date of publication of application: 28.12.2011
(73) Proprietor: Versitech Limited, Hong Kong (CN); City University of Hong Kong, Hong Kong (CN)
(72) Inventor: YEUNG, Waikwok, Kelvin, Hong Kong (CN); CHEUNG, Manchee, Hong Kong (CN); LUK, Dipkei, Keith, Hong Kong (CN); YEUNG, Cheyan, Hong Kong (CN); KAO, Yitsun, Richard, Hong Kong (CN); CHU, Kimho, Paul, Hong Kong (CN)
(74) Representative: Epping - Hermann - Fischer
(86) International application number: PCT/CN2010/000216
(87) International publication number: WO 2010/094212

(56) References cited:
- WO-A2-2008/085578
- CN-A- 1 933 859
- CN-A- 101 020 087
- CN-A- 101 128 224
- MANDL, S. ET AL.: 'Investigation on plasma immersion ion implantation treated medical implants.' BIOMOLECULAR ENGINEERING. vol. 19, 2002, pages 129 - 132, XP004378082
- N.D. RAWLINGS ET AL.: "MEROPS: the peptide database", NUCLEIC ACIDS RESEARCH, vol. 34, 1 January 2006 (2006-01-01), pages D270-D272, DOI: 10.1093/nar/gkj089
- D.L. Nelson, M.M. Cox: "Lehninger Principles of Biochemistry, 4th Ed.", pages 99-100,

## Description

### BACKGROUND OF THE INVENTION

### Field of the Invention

The present invention relates to controllable antimicrobial and biocompatible titanium alloys for reducing post-operative implant-related bacterial infections. The present invention further relates to surface techniques and their mechanisms for modifying material surfaces so as to enhance the surface antimicrobial properties and achieve controllable release of antimicrobial peptides.

### Description of the Related Art

Biomaterials such as stainless steel and titanium alloys are widely used in orthopaedic and dental surgery. Internal bone fracture fixations, spinal deformity corrections and total joint replacements are commonly seen in orthopaedic procedures in which metallic biomaterials are usually used to maintain biomechanical integrity. However, adhesion and proliferation of microorganisms on these implants may induce post-operative microbial infections. In addition, they may result in implant loosening or other serious complications. According to R.O. Darouiche in "Treatment of Infections Associated with Surgical Implants" (The New England Journal of Medicine 2004;350(14):1422-1429), two to five percent of the patients receiving orthopaedic procedures may be required to undergo revision surgery to remove or replace the infected implants. In addition to the increase in cost to the public healthcare system, the morbidity and mortality rates are undoubtedly increased.

Surface modifications have been proposed to modify the surface properties of biomaterials so as to maintain their bulk mechanical properties after treatment. To reduce bacterial adhesion and proliferation, certain surface treatments such as antibiotic, silver and copper ions loaded surfaces have been studied. The fabrication of the surface treated implants have been carried out by the sol-gel technique, physical vapor deposition (PVD), chemical vapor deposition (CVD), immersion and ion implantation. Unfortunately, the release rate of those surface treatment deposits is difficult to manipulate by using the current technologies. In addition, as shown by S-H. Shin et al. in "The effects of nano-silver on the proliferation and cytokine expression by peripheral blood mononuclear cells" (International Immunopharmacology 2007;7(13):1813-1818) and B.S. Atiyeh et al. in "Effect of silver on burn wound infection control and healing: Review of the literature" (Burns 2007;33(2): 139-148), silver and copper ions may poison the surrounding human cells.

Previous studies have also involved the loading of antibiotics such as vancomycin onto a titanium substrate surface via the sol-gel technique. One obstacle to prevalent use of this technique is the uncontrollable degradation of antibiotics once exposed to body fluid. This effect is shown in "Controlled release of vancomycin from thin sol-gel films on titanium alloy fracture plate material" by S. Radin et al. (Biomaterials 2007;28(9):1721-1729) and "In vivo tissue response to resorbable silica xerogels as controlled-release materials" by S. Radin et al. (Biomaterials 2005;26(9):1043-1052). Accordingly, the release rate of the antibiotic is difficult to control with the use of existing techniques. Clinically, deep wound infection occurring after period of operation is not uncommonly seen. The conventional antibiotics coating is not able to tackle this complication due to the degradation over time of the antibiotics. The unprotected surface of the implant is then exposed to the invading bacteria. Moreover, the uncontrollable release of the antibiotics may occur in excess and thus spoil the normal function of the peripheral cells.

Thus, there continues to exist a need in the art for implantable biomaterials with surface antimicrobial properties.

### BRIEF SUMMARY OF THE INVENTION

The present invention provides surgical implants as defined in claim 1. In a specific embodiment, the implantable titanium alloys are fabricated by using plasma immersion ion implantation and deposition (PIII&D), pressurized hydrothermal treatment, and antimicrobial peptide coating. Advantageously, these specially modified titanium alloys are able to suppress the adhesion and proliferation of microorganisms.

Embodiments of the present invention involve performing surface techniques to modify the surface of a material in order to form one or several antimicrobial layers capable of resisting microbial adhesion and proliferation.

In one embodiment, the surface modification techniques include PIII&D and/or a pressurized hydrothermal treatment. In a further embodiment, a surface layer is fabricated by using a technique for the immobilization of controllable release antimicrobial peptides. According to embodiments, an assembly of surface modification techniques, or a combination of one or more of the technique(s) disclosed herein and one or more of the existing technique(s) such as antibiotics coating or silver doping can be used to fabricate the surface layer(s).

According to one embodiment, the surface modification by using PIII&D can implant various sources including ions, electrons, free radicals, atoms and molecules onto the substrate surface as the basal surface to inhibit microbial adhesion and growth. This basal surface is able to resist the attachment of microorganisms and the formation of biofilm. However, advantageously, this surface allows the attachment and growth of mammalian cells.

In a further embodiment, reactive functional groups such as -OH and -NH₂ groups are incorporated onto the basal surface, forming an intermediate surface layer to facilitate a linker molecule's coupling, for example, a linker such as 3-aminopropyltriethoxysilane (APTES). The fabrication of the reactive functional groups can be performed through a pressurized hydrothermal treatment or another PIII&D process. The antimicrobial peptides will then aggregate on the outermost layer of the titanium substrate to form an antimicrobial peptide coating.

The subject titanium alloy modified by PIII&D treatment has the ability to resist bacterial adhesion and growth, and its modified surface is also compatible with mammalian cells.

The release of antimicrobial peptides from the outermost layer is only triggered when bacteria start to attach to the titanium surface, and is thus self-controllable. In contrast, for conventional antibiotic or antimicrobial peptide coatings, the conventional release mechanism of the antibacterial peptide or antibiotics loaded surfaces is subject to the exposure to the body fluids and therefore uncontrollable.

The present invention can be applied to orthopaedic implants, but embodiments are not limited thereto. For example, the present invention can be applied to all implantable biomaterials such as cardiovascular implants and dental implants.

### BRIEF DESCRIPTION OF THE DRAWINGS

The patent or application file contains at least one drawing executed in color. Copies of this patent or patent application publication with color drawing(s) will be provided by the Office upon request and payment of the necessary fee.

The detailed description of the present invention will be better understood in conjunction with the accompanying drawings, wherein like reference characters represent like elements, as follows:
Figure 1 is a table providing the PIII&D surface treatment conditions of titanium alloy samples.
Figure 2A is an XPS elemental depth profile of an untreated titanium alloy control.
Figure 2B is an XPS elemental depth profile of a 50 Hz oxygen PIII&D treated titanium alloy sample fabricated in accordance with an embodiment of the present invention.
Figure 2C is an XPS elemental depth profile of a 100 Hz oxygen PIII&D treated titanium alloy sample fabricated in accordance with an embodiment of the present invention.
Figure 3A is an image of the AFM surface topography on the untreated titanium alloy control.
Figure 3B is an image of the AFM surface topography on a 50 Hz oxygen PIII&D treated titanium alloy sample fabricated in accordance with an embodiment of the present invention.
Figure 3C is an image of the AFM surface topography on a 100 Hz oxygen PIII&D treated titanium alloy sample fabricated in accordance with an embodiment of the present invention.
Figure 4 is a graph showing number of adhered bacteria *Staphylococcus aureus* on the untreated titanium alloy and 50Hz and 100Hz oxygen PIII&D treated titanium alloy samples after one hour of bacteria culturing.
Figure 5A is a fluorescent micrograph of an untreated control titanium alloy sample. The green marks represent live *Staphylococcus aureus.*
Figure 5B is the fluorescent micrograph of a 50 Hz oxygen PIII&D treated titanium alloy sample fabricated in accordance with an embodiment of the present invention. The green and red marks represent live and dead *Staphylococcus aureus* respectively.
Figure 5C is the fluorescent micrograph of a 100 Hz oxygen PIII&D treated titanium alloy sample fabricated in accordance with an embodiment of the present invention. The green and red marks represent live and dead *Staphylococcus aureus* respectively.
Figure 6A is a fluorescent micrograph of an untreated control titanium alloy sample. The green marks represent live mammalian cells of EGFP-expressing mouse osteoblasts.
Figure 6B is the fluorescent micrograph of a 50 Hz oxygen PIII&D treated titanium alloy sample fabricated in accordance with an embodiment of the present invention. The green marks represent live mammalian cells of EGFP-expressing mouse osteoblasts.
Figure 6C is the fluorescent micrograph of a 100 Hz oxygen PIII&D treated titanium alloy sample fabricated in accordance with an embodiment of the present invention. The green marks represent live mammalian cells of EGFP-expressing mouse osteoblasts.
Figure 7 is an illustration of the controllable release mechanism utilized in an embodiment of the present invention. The bacterial protease cleaves the peptide bonding at the C-terminal side of glutamic acid and allows the release of antibacterial peptide.
Figure 8A is a titanium alloy implant incorporated with an antibacterial peptide through linker molecules such as APTES.
Figure 8B is the chemical structure of a linker molecule APTES.
Figure 8C is the chemical structure of an antibacterial peptide for immobilization on the titanium alloy implant. This is an example peptide that is incorporated onto the outermost layer of an implantable metallic surface according to an embodiment of the present invention.
Figure 9 is an illustration of the fabrication process of antibacterial layers on the substrate surface with the application of grid. The grid with longitudinal lines is placed to cover the substrate surface (on the left). After the application of PIII&D, lines of the antibacterial layer are formed (on the right). By shifting the grid, alternate lines of antibacterial basal layer and peptide layer can be obtained (the order of applying PIII&D and peptide can be reversed).

### DETAILED DISCLOSURE OF THE INVENTION

Embodiments of the present invention provide one or more antimicrobial layers capable of resisting microbial adhesion and proliferation on a material surface. According to implementations of the present invention, the surface techniques of plasma immersion ion implantation and deposition (PIII&D) and pressurized hydrothermal treatment, and the methods of immobilizing antimicrobial peptides onto the material surfaces are used to enhance the antimicrobial properties of a material surface.

To reduce and potentially eliminate the technical complications of existing antimicrobial techniques, the fabricating of the antimicrobial surface with the use of PIII&D, pressurized hydrothermal treatment, and antimicrobial peptide coating as described in embodiments herein, provides an alternative to suppress the adhesion and proliferation of microorganisms.

Embodiments of the present invention provide a controllable antimicrobial and biocompatible Ti alloy that can resist bacterial adhesion and proliferation without compromising its biocompatibility and original mechanical properties. The controllable antimicrobial and biocompatible Ti alloy of embodiments of the present invention can resist microbial adhesion and proliferation by using surface techniques including PIII&D, intermediate linker formation, and the immobilization of controllable release antimicrobial peptides on the outmost surface. The medical applications of this special titanium alloy can include but are not limit to orthopaedic surgeries, cardiovascular, and dental operations.

For PIII&D, various sources including ions, electrons, free radicals, atoms and molecules can be implanted onto the substrate surface as the basal surface to protect against microbial adhesion and growth. This basal surface is able to resist the attachment of microorganisms and the formation of biofilm.

For the pressurized hydrothermal treatment, functional groups of interest can be incorporated onto the substrate surface as another basal surface to resist microbial adhesion and proliferation.

The second layer next to or on top of the aforementioned basal surface can be fabricated and covered by antimicrobial peptides. The antimicrobial peptide coating formed in accordance with the present invention performs such that the release of antimicrobial peptide is triggered when the microorganisms start to attach to the surface. In contrast, the conventional release mechanism of antibiotics loaded surfaces are subject to the exposure to the body fluids, and are therefore uncontrollable. According to an embodiment, the antimicrobial peptide is capable of performing differently than the conventional antibiotics based on a controllable manner in which the antimicrobial peptides will be cleaved off from the surface by the proteins or enzymes secreted by those incoming microorganisms. The released peptides can then terminate the incoming microorganisms. This defense mechanism is comparatively long lasting and more stable than the conventional approaches. This combined mechanism can be applied to various medical implants made of metals, alloys, ceramics, polymers, composites and hybrid materials, and even the medical implants with irregular shapes.

In accordance with embodiments, implantable Ti alloys are provided with controllable antimicrobial and biocompatible properties for reducing post-operative implant-related bacterial infections. Certain embodiments having these controllable properties can resist infections both short term and long term (6 months or more) post-operatively.

It should be noted that when the substrate material and/or location of application are altered, an optional optimization of the sources and/or parameters of the surface techniques can be performed. For instance, an optimization of the plasma source, application frequency, voltage and duration of implantation can be involved when applying the PIII&D technique. Furthermore, the antimicrobial ability of implantable titanium alloy may involve an optimization of the sources and/or parameters of PIII&D, e.g. plasma source, application frequency, voltage, and duration of implantation. The parameters of the PIII&D include using a plasma source of at least one of water, oxygen, ammonia, fluorine, nitrogen, gold, silver, copper, silicon-carbine, iridium oxide, carbon, and diamond like carbon; an implantation voltage in a range of 1kV - 100kV; a frequency in a range of 1 Hz - 1,000Hz; and a period of time in a range of 1 min - 100 hours.

In another embodiment, an optimization of the composition, sequence and amount of the antimicrobial peptide and the species of linker molecules connecting antimicrobial peptide and the substrate can be involved when immobilizing the peptide on the material surface. For example, the composition, sequence and amount of the antimicrobial peptide and the species of linker molecules connecting antimicrobial peptide and the titanium substrate may also alter its antimicrobial ability when immobilizing the peptides on the material surface. Thermal treatment and/or radiation treatment to the biomaterials such as the titanium alloy can further be involved during the fabrication process so as to enhance the antimicrobial properties of these surface layers and alloys.

In order to better understand the present invention, a titanium alloy treated by PIII&D oxygen will be used as one of examples to highlight its antibacterial properties and biocompatibility.

### Antimicrobial Basal Layer Fabricated by PIII&D

An antimicrobial basal layer on an implantable titanium alloy can be fabricated by incorporating ions, electrons, free radicals, atoms and molecules onto its surface using, for example, oxygen, water, ammonia, fluorine, nitrogen, gold, silicon-carbide, iridium oxide, carbon or diamond-like carbon. The techniques used to modify the surface of the implant can involve PIII&D and/or other treatments such as a pressurized hydrothermal treatment. In general, the newly-formed layer(s) possesses antimicrobial properties and biocompatibility, thereby protecting the metallic surface of the implant from microbial attack and accommodating for the growth of mammalian cells.

In an embodiment of the titanium alloy treated by oxygen PIII&D, the material surface of the titanium alloy implant is surrounded by high density oxygen plasma and biased to a high negative potential relative to the chamber in which the oxygen plasma source is generated by a high voltage AC/DC supply. The oxygen plasma ions are accelerated across the sheath formed around the substrate (titanium alloy implant) by an electromagnetic field and implanted onto the surface of the substrate (titanium alloy implant). Both the dose of the implanted ions and the depth of the implanted layer can be optimized by adjusting the plasma density, pulse width, applied voltage and repetition frequency in order to enhance the antimicrobial property.

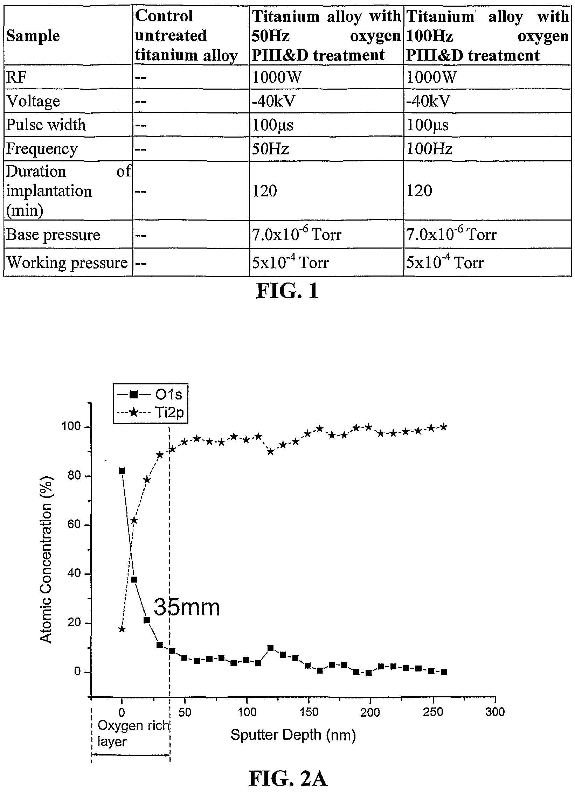

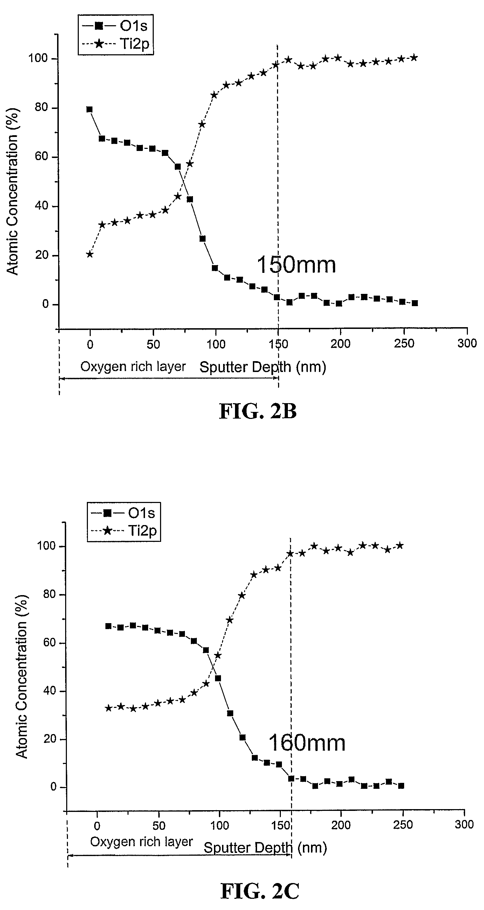

According to an example implementation providing surface chemical characteristics of the oxygen PIII&D treated titanium alloys, the elemental depth profile is investigated by X-ray photoelectron spectroscopy (XPS) so as to reveal the surface composition of the samples. For these samples, polished and cleaned medical grade titanium Ti-6A1-4V discs with 5 mm diameter and 1 mm thickness were implanted with oxygen plasma using a PIII&D technique in accordance with an embodiment of the present invention. Fig. 1 shows a Table indicating the treatment conditions of the embodying oxygen PIII&D samples described in this disclosure, while Fig. 2A shows the results of an untreated titanium sample, Fig. 2B shows the chemical profiles of the 50 Hz oxygen treated samples, and Fig. 2C shows the chemical profile of the 100 Hz oxygen treated samples. It can be observed from the figures that the oxygen content has been enriched at the Ti alloy surface. In addition, the thickness of the oxygen rich layer can be increased with the implantation frequency.

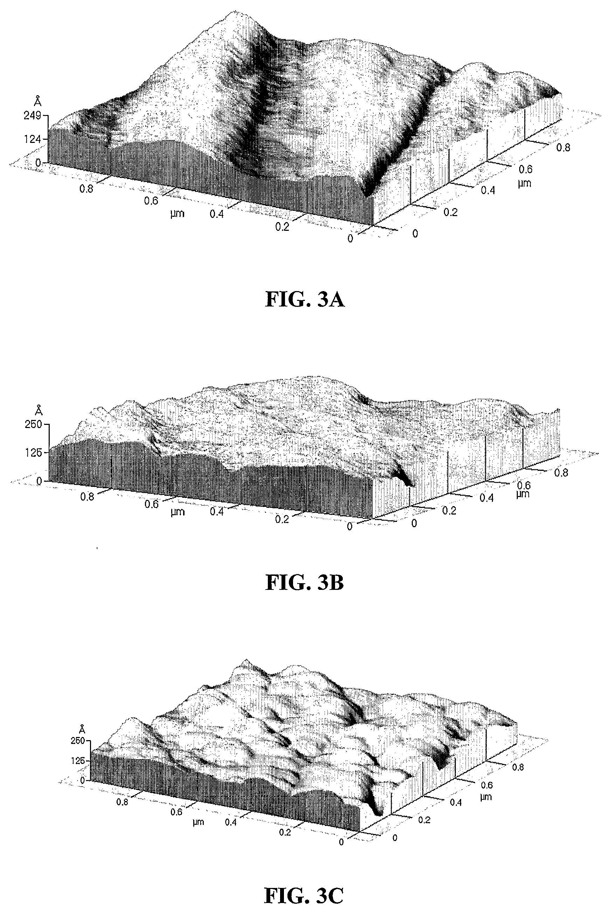

In the same example implementation, the treated titanium surface topography and roughness is evaluated by atomic force microscopy (AFM). The results of the untreated and treated samples are shown in Figs. 3A-3C. The surface roughness can be indicated by the root mean square (RMS) value of the AFM surface topography plot of the samples. The RMS of the control is 40 Å, the RMS of the 50 Hz oxygen treated sample is 22 Å, and the RMS of the 100 Hz oxygen treated sample is 24 Å. As indicated by Figs. 3A-3C, it can be seen that the treated surfaces are smoothened as compared to the non-treated surface. In particular, the roughness of the titanium surface was reduced after treatment. In addition, a specific surface pattern (ripple-like pattern) appeared on the titanium surface after it was modified by the oxygen PIII&D. This ripple-like pattern can help reduce bacterial adhesion and attachment.

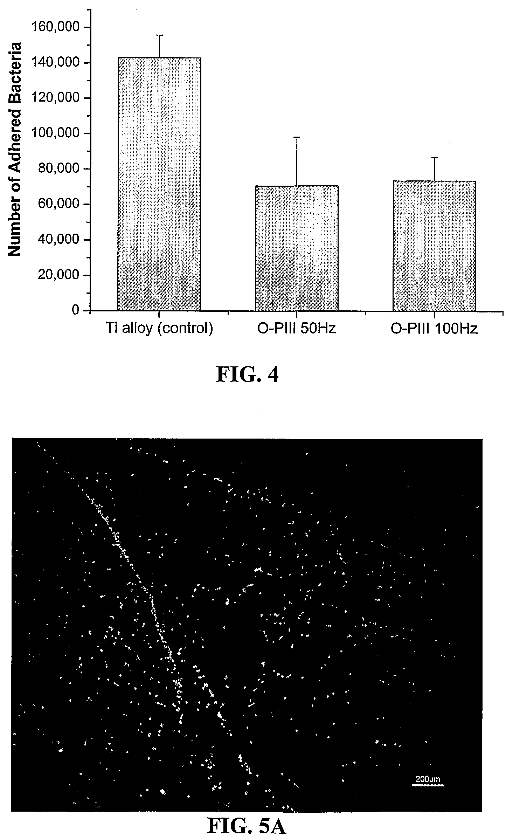

The antibacterial/antimicrobial properties of the specially treated titanium alloys in accordance with the present invention have been characterized by standard colony forming unit (CFU) counting with *Staphylococcus aureus,* which is commonly seen in orthopaedic implant-related infection. The *Staphylococcus aureus* adhered on the metallic surfaces was detached by sonication. The CFU of the detached bacteria suspension is determined by surface plating on Brain Heart Infusion (BHI) agar after being incubated at 37°C for 24 hrs. As demonstrated in the graph of Fig. 4, the reduction of bacterial adhesion on treated titanium surfaces is significant after the PIII&D surface treatments in accordance with the present invention.

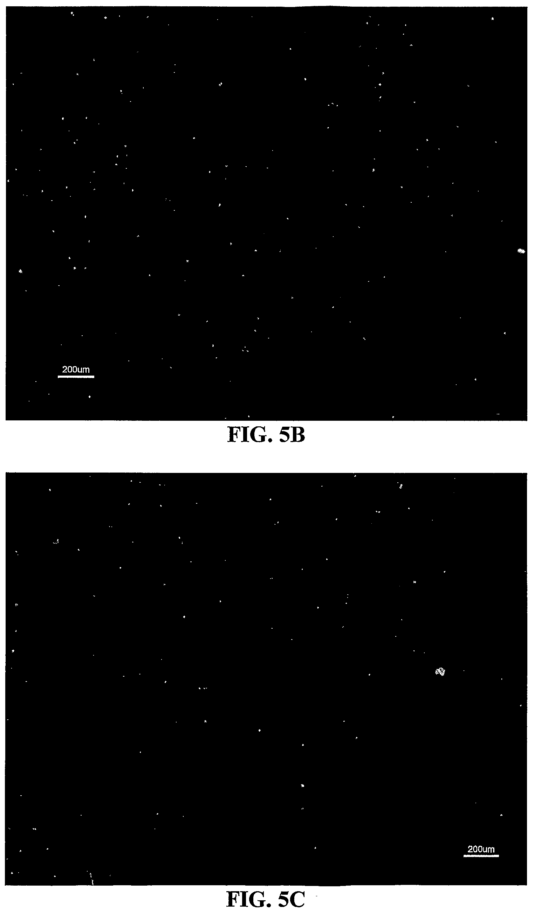

Antibacterial/antimicrobial properties of the treated titanium surfaces can also be visualized by applying fluorescent microscopy. Figs. 5A-5C show the morphology of adhered bacteria on the untreated and treated titanium alloys. It can be easily observed by comparing Fig. 5A to Figs. 5B and 5C that the titanium alloy treated with oxygen PIII&D successfully inhibits the adhesion of *Staphylococcus aureus.*

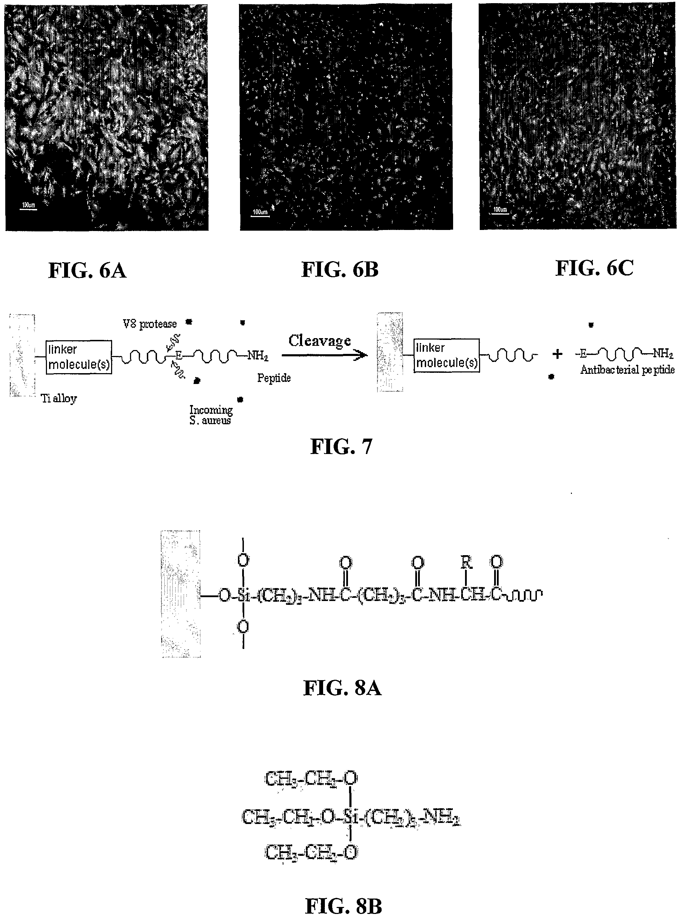

Furthermore, with respect to the biocompatibility of treated titanium alloys in accordance with embodiments of the present invention, mammalian cell culturing using EGFP-expressing mouse osteoblasts have been conducted and are visualized by applying fluorescent microscopy. Figs. 6A-6C show the morphology of adhered mammalian cells on the untreated and treated titanium alloys. The results revealed that the titanium surfaces treated with 50 Hz and 100 Hz oxygen plasma treatment (Figs. 6B and 6C, respectively) are well tolerated by mammalian cells as compared with the untreated titanium (Fig. 6 A) after 3 days of culturing.

In another embodiment, a pressurized hydrothermal treatment can be applied to establish reactive functional groups exhibiting antimicrobial functions. For example, implantable titanium alloy with other reactive functional groups such as -O_{X}H_{Y} (where x and y are natural numbers) exhibiting antimicrobial functions can be achieved by the pressurized hydrothermal treatment. In yet another embodiment, PIII&D can be used to form a reactive functional amide group (for example -NH₂) on the titanium alloy surface. For example, nitrogen, oxygen, and ammonia PIII&D can be performed under various conditions (including concentration of implanted elements, implantation voltage and frequency, treatment time, and temperature) to form the reactive functional amide groups on the titanium alloy surface.

However, the reactive function group layer is not limited to establishing antimicrobial functional groups. For example, these reactive functional groups can be regarded as an intermediate surface to facilitate the formation of linker molecules such as APTES. Then, the linker molecules can be used to aggregate external antimicrobial molecules such as chitosan and antimicrobial peptides on the surface of implantable titanium alloys. This mechanism is discussed in the example below.

### Controllable Release Antimicrobial Peptide

A titanium alloy having a layer of antimicrobial peptide can be fabricated on the substrate surfaces alone, next to or on top of the aforementioned basal layer, or in a combination with other existing antimicrobial surface techniques such as coating and plasma spraying. For example, an implantable titanium alloy with a coating of controllable release antimicrobial peptide on its outermost surface can be fabricated by surface treatments such as conventional coating and plasma spraying.

The antimicrobial peptide on the plasma treated titanium alloy according to an embodiment is fabricated to provide a controllable manner in which the antimicrobial peptides will be cleaved off from the substrate surface by the proteins or enzymes secreted by those incoming microorganisms.

The subject implantable titanium alloy with antimicrobial peptide coating possesses a manner of controllable release. The antimicrobial peptides will be cleaved off from the titanium surface when the proteins or enzymes secreted by the particular incoming bacteria as the bacterial approach the implant. The released peptides can then terminate the incoming microorganisms. In an embodiment of antibacterial peptide immobilized titanium alloy orthopaedic implants, the V8 protease secreted by the *Staphylococcus aureus* cleaves the peptide bonding of the antibacterial peptide at the C-terminal side of glutamic acid. This mechanism is demonstrated in Fig. 7. The controllable release mechanism of antibacterial peptides can be applied to orthopaedic, dental and cardiovascular implants so as to work against bacteria such as *Staphylococcus aureus, Pseudomonas aeruginosa, Staphylococcus epidermidis*, *Salmonella Dublin, Escherichia coli, Porphyromonas gingivalis,* and *Streptococcus mutans,* etc.

In order to immobilize the antimicrobial peptides onto the titanium surfaces, reactive functional groups and linkers are preferably incorporated to the metallic substrate first so as to facilitate the subsequent peptide attachment. As mentioned above, reactive functional groups fabricated on the titanium substrate surfaces by pressurized hydrothermal treatment (or PIII&D) may also contain antimicrobial functions. Alternatively, these functional groups can also be regarded as an intermediate surface to facilitate the formation of linker molecules such as APTES such that the antimicrobial substances such as chitosan and antibacterial peptides can aggregate on the surface of implantable titanium alloys.

In an embodiment, the implantable titanium alloy with a layer of hydroxide functional groups (-OH) can be established by submerging the metal (titanium alloy) into a hydrogen peroxide solution followed by a series of heating processes under pressurized conditions. A second reagent such as concentrated sodium hydroxide solution may be used to enhance the yield of -OH functional groups. For those metallic surfaces with -OH functional groups after pressurized hydrogen peroxide treatment, the antibacterial/antimicrobial peptides are able to covalently attach to the -OH groups on the titanium alloy substrate surface by applying coupling agents (linkers). For instance, 3-aminopropyltriethoxysilane (APTES) can be used as one of the coupling agents. One end of the APTES linker can be attached to the -OH group on the titanium alloy substrate surface and the other end with a primary amine group can be linked up with other linkers and/or antibacterial/antimicrobial peptides. The amine group at the APTES linker can be easily converted into other functional groups such as COOH by various chemical reactions such as oxidation and carbonation.

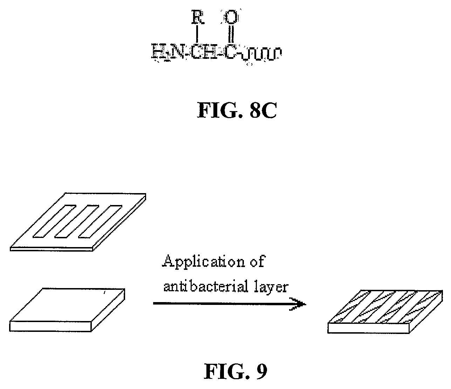

Figs. 8A-8C illustrate the attachment of an antibacterial/antimicrobial peptide to the titanium alloy in which its surface is firstly treated with pressurized hydrothermal treatment and followed by the attachment of APTES linkers and glutaric anhydride. The antibacterial/antimicrobial peptide can be subsequently incorporated to the free end of the APTES linker or glutaric anhydride. Fig. 8A shows the chemical structure of the embodiment of an antibacterial/antimicrobial peptide immobilized on a titanium alloy implant surface. Figs. 8B and 8C show the chemical structure of APTES molecule and antibacterial peptide, respectively.

During the fabrication process, particular grids (or masks) can be applied to cover partial areas of the substrate so as to establish various patterns over the metallic substrate surface, thereby forming specific patterns of antibacterial/antimicrobial peptides and surfaces on the titanium alloy as shown in Fig. 9. In an embodiment, if the grid with longitudinal lines is applied throughout the fabrication process, alternate lines of antibacterial basal layer and peptide layer on the substrate surface can be resulted.

## Claims

1. A surgical implant comprising:
a titanium alloy modified by a plasma treatment and having a controllable release antimicrobial peptide coating disposed on the surface of the modified titanium alloy,
wherein the controllable release antimicrobial peptide coating has a controllable release mechanism for the antimicrobial peptide that is triggered by an incoming bacterial attack, wherein the controllable release antimicrobial peptide coating includes stable antimicrobial peptides having a cleavable mechanism that is triggered by protease released by incoming bacteria,
and wherein the surface of the modified titanium alloy is compatible with mammalian cells.

2. The surgical implant according to claim 1, wherein the modified titanium alloy is able to resist bacterial attachment and growth on its surface.

3. The surgical implant according to claim 1, wherein original mechanical properties of the titanium alloy are not altered by the plasma treatment and antimicrobial peptide coating.

4. The surgical implant according to claim 1, wherein the modified titanium alloy is configured for implantation in orthopaedic, cardiovascular, or dental operations.

5. The surgical implant according to claim 1, wherein the plasma treatment process involves plasma immersion ion implantation and deposition (PIII&D), and the parameters of the PIII&D include a plasma source of at least one of water, oxygen, ammonia, fluorine, nitrogen, gold, silver, copper, silicon-carbine, iridium oxide, carbon, and diamond like carbon, an implantation voltage in a range of 1kV - 100kV, a frequency in a range of 1 Hz - 1,000Hz, and duration of time in a range of 1 min - 100 hours.

6. The surgical implant according to claim 1, wherein the modified titanium alloy has a surface topography of a ripple-like pattern.

7. The surgical implant according to claim 1, wherein the controllable release antimicrobial peptide coating is effective against at least one of *Staphylococcus aureus, Pseudomonas aeruginosa, Staphylococcus epidermidis, Salmonella Dublin, Escherichia coli, Porphyromonas gingivalis,* and *Streptococcus mutans.*

8. The surgical implant according to claim 1, wherein the antimicrobial peptides of the controllable release antimicrobial peptide coating include anionic peptides, cationic peptides, anionic and cationic peptides that contain cysteine and form disulfide bonds, or catioinic peptides enriched for specific amino acid attachment.

9. The surgical implant according to claim 1, wherein the modified titanium alloy is capable of withstanding short-term and long-term deep tissue infection.

10. The surgical implant according to claim 1, further comprising linker molecules between the plasma treated titanium alloy surface and the antimicrobial peptide coating, preferably comprising 3-aminopropyltriethoxysilane (APTES).

11. The surgical implant according to claim 10, wherein release of antimicrobial peptides from the controllable release antimicrobial peptide coating is triggered by breakdown of the linker molecules, wherein the breakdown of linker molecules is triggered by incoming bacteria.

12. The surgical implant according to claim 10, further comprising an intermediate layer formed by reactive functional groups, preferably comprising an -OH group, wherein the -OH group is fabricated by pressurized hydrogen peroxide treatment and a series of heating processes or an amide group (-NH₂), wherein the amide group is fabricated by nitrogen, oxygen and ammonia plasma immersion ion implantation, on the plasma treated titanium alloy surface, wherein the intermediate layer facilitates linker molecule coupling on the plasma treated titanium alloy surface and facilitates antimicrobial peptide aggregation when forming the controllable release antimicrobial peptide coating.

13. The surgical implant according to claim 12, wherein the reactive functional group includes an -OH group, wherein the linker molecule includes APTES, wherein the APTES attaches at one end to the -OH group on the plasma treated titanium surface, wherein another end of the APTES having a primary amine group links up with additional linkers and/or antimicrobial peptides and the primary amine group at the another end of the APTES is converted into a second functional group by a series of chemical reactions.

## Patentansprüche

1. Chirurgisches Implantat, umfassend:
eine Titanlegierung, die durch eine Plasmabehandlung modifiziert worden ist, und die eine antimikrobielle Peptidbeschichtung zur kontrollierbaren Freisetzung aufweist, die auf der Oberfläche der modifizierten Titanlegierung angeordnet ist,
wobei die antimikrobielle Peptidbeschichtung zur kontrollierbaren Freisetzung einen kontrollierbaren Freisetzungsmechanismus für das antimikrobielle Peptid aufweist, der durch einen ankommenden Bakterienbefall ausgelöst wird, wobei die antimikrobielle Peptidbeschichtung zur kontrollierbaren Freisetzung stabile antimikrobielle Peptide beinhaltet, mit einem spaltbaren Mechanismus, der durch Protease ausgelöst wird, die durch ankommende Bakterien freigesetzt wird,
und wobei die Oberfläche der modifizierten Titanlegierung mit Säugetierzellen kompatibel ist.

2. Chirurgisches Implantat nach Anspruch 1, wobei die modifizierte Titanlegierung einer bakteriellen Anhaftung und dem Wachstum auf ihrer Oberfläche widerstehen kann.

3. Chirurgisches Implantat nach Anspruch 1, wobei die ursprünglichen mechanischen Eigenschaften der Titanlegierung durch die Plasmabehandlung und die antimikrobielle Peptidbeschichtung nicht verändert werden.

4. Chirurgisches Implantat nach Anspruch 1, wobei die modifizierte Titanlegierung für die Implantation bei orthopädischen, kardiovaskulären oder Zahnoperationen konfiguriert ist.

5. Chirurgisches Implantat nach Anspruch 1, wobei das Plasmabehandlungsverfahren Plasma-Immersions-Ionenimplantation und Abscheidung (PIII&D) beinhaltet, und die Parameter von PIII&D eine Plasmaquelle von mindestens einem von Wasser, Sauerstoff, Ammoniak, Fluor, Stickstoff, Gold, Silber, Kupfer, Siliciumcarbid, Iridiumoxid, Kohlenstoff und diamantartigem Kohlenstoff, eine Implantationsspannung in einem Bereich von 1kV - 100kV, eine Frequenz in einem Bereich von 1 Hz - 1000 Hz und eine Zeitdauer in einem Bereich von 1 min - 100 Stunden umfassen.

6. Chirurgisches Implantat nach Anspruch 1, wobei die modifizierte Titanlegierung eine Oberflächentopographie eines wellenartigen Musters aufweist.

7. Chirurgisches Implantat nach Anspruch 1, wobei die antimikrobielle Peptidbeschichtung zur kontrollierbaren Freisetzung gegen mindestens einen Mikroorganismus von *Staphylococcus aureus, Pseudomonas aeruginosa, Staphylococcus epidermidis, Salmonella Dublin, Escherichia coli, Porphyromonas gingivalis* und *Streptococcus mutans* wirksam ist.

8. Chirurgisches Implantat nach Anspruch 1, wobei die antimikrobiellen Peptide der antimikrobiellen Peptidbeschichtung zur kontrollierbaren Freisetzung anionische Peptide, kationische Peptide, anionische und kationische Peptide, die Cystein enthalten und Disulfidbindungen bilden, oder kationische Peptide, die in Bezug auf spezifische Aminosäurebindung angereichert sind, umfassen.

9. Chirurgisches Implantat nach Anspruch 1, wobei die modifizierte Titanlegierung kurzfristiger und langfristiger Tiefengewebe-Infektion standhalten kann.

10. Chirurgisches Implantat nach Anspruch 1, das zudem Linkermoleküle zwischen der plasmabehandelten Titanlegierungs-Oberfläche und der antimikrobiellen Peptidbeschichtung umfasst, die vorzugsweise 3-Aminopropyltriethoxysilan (APTES) umfassen.

11. Chirurgisches Implantat nach Anspruch 10, wobei die Freisetzung von antimikrobiellen Peptiden aus der antimikrobiellen Peptidbeschichtung zur kontrollierbaren Freisetzung durch Abbau der Linkermoleküle ausgelöst wird, wobei der Abbau der Linkermoleküle durch ankommende Bakterien ausgelöst wird.

12. Chirurgisches Implantat nach Anspruch 10, zudem umfassend eine durch reaktive funktionelle Gruppen gebildete Zwischenschicht, die vorzugsweise eine -OH-Gruppe, wobei die -OH-Gruppe durch Behandlung mit unter Druck stehendem Wasserstoffperoxid und eine Reihe von Erhitzungsverfahren erzeugt wird, oder eine Amidgruppe (-NH₂) enthält, wobei die Amidgruppe durch Stickstoff-, Sauerstoff- und Ammoniak-Plasma-Immersions-Ionenimplantation auf der plasmabehandelten Titanlegierungsoberfläche erzeugt wird, wobei die Zwischenschicht die Linkermolekülkopplung auf der plasmabehandelten Titanlegierungsoberfläche erleichtert und die antimikrobielle Peptidaggregation bei der Bildung der antimikrobiellen Peptidbeschichtung zur kontrollierbaren Freisetzung erleichtert.

13. Chirurgisches Implantat nach Anspruch 12, wobei die reaktive funktionelle Gruppe eine -OH-Gruppe enthält, wobei das Linkermolekül APTES umfasst, wobei APTES an einem Ende an die -OH-Gruppe auf der plasmabehandelten Titanoberfläche gebunden ist, wobei das andere Ende von APTES, das eine primäre Amingruppe hat, mit zusätzlichen Linkern und/oder antimikrobiellen Peptiden verbunden ist, und die primäre Amingruppe am anderen Ende von APTES durch eine Reihe von chemischen Reaktionen in eine zweite funktionelle Gruppe umgewandelt wird.

## Revendications

1. Implant chirurgical comprenant :
un alliage de titane modifié par un traitement par plasma et ayant un revêtement de peptides antimicrobiens à libération contrôlable disposé sur la surface de l'alliage de titane modifié,
le revêtement de peptides antimicrobiens à libération contrôlable ayant un mécanisme de libération contrôlable pour le peptide antimicrobien qui est déclenché par une attaque bactérienne entrante, le revêtement de peptides antimicrobiens à libération contrôlable comportant des peptides antimicrobiens stables ayant un mécanisme de clivage qui est déclenché par une protéase libérée par des bactéries entrantes,
et la surface de l'alliage de titane modifié étant compatible avec les cellules de mammifères.

2. Implant chirurgical selon la revendication 1, dans lequel l'alliage de titane modifié est capable de résister à la fixation et la croissance de bactéries sur sa surface.

3. Implant chirurgical selon la revendication 1, dans lequel les propriétés mécaniques initiales de l'alliage de titane ne sont pas modifiées par le traitement par plasma et le revêtement de peptides antimicrobiens.

4. Implant chirurgical selon la revendication 1, dans lequel l'alliage de titane modifié est configuré pour une implantation dans des opérations orthopédiques, cardiovasculaires, ou dentaires.

5. Implant chirurgical selon la revendication 1, dans lequel le procédé de traitement par plasma implique l'implantation et le dépôt d'ions par immersion dans un plasma (PIII&D), et les paramètres du PIII&D comportent une source de plasma d'au moins un élément parmi l'eau, l'oxygène, l'ammoniac, le fluor, l'azote, l'or, l'argent, le cuivre, le carbure de silicium, l'oxyde d'iridium, le carbone et le carbone diamant, une tension d'implantation dans une gamme de 1 kV - 100 kV, une fréquence dans une gamme de 1 Hz - 1000 Hz, et une durée dans une gamme de 1 min - 100 heures.

6. Implant chirurgical selon la revendication 1, dans lequel l'alliage de titane modifié a une topographie de surface d'un motif en forme d'ondulations.

7. Implant chirurgical selon la revendication 1, dans lequel le revêtement de peptides antimicrobiens à libération contrôlable est efficace contre au moins une bactérie parmi *Staphylococcus aureus, Pseudomonas aeruginosa, Staphylococcus epidermidis, Salmonella Dublin, Escherichia coli, Porphyromonas gingivalis,* et *Streptococcus mutans.*

8. Implant chirurgical selon la revendication 1, dans lequel les peptides antimicrobiens du revêtement de peptides antimicrobiens à libération contrôlable comprennent des peptides anioniques, des peptides cationiques, des peptides anioniques et cationiques qui contiennent de la cystéine et forment des liaisons disulfure, ou des petites cationiques enrichis pour la fixation d'un acide aminé spécifique.

9. Implant chirurgical selon la revendication 1, dans lequel l'alliage de titane modifié est capable de supporter une infection tissulaire profonde à court terme et à long terme.

10. Implant chirurgical selon la revendication 1, comprenant en outre des molécules de liaison entre la surface de l'alliage de titane traité par plasma et le revêtement de peptides antimicrobiens, comprenant de préférence du 3-aminopropyltriéthoxysilane (APTES).

11. Implant chirurgical selon la revendication 10, dans lequel la libération de peptides antimicrobiens du revêtement de peptides antimicrobiens à libération contrôlable est déclenchée par la rupture des molécules de liaison, la rupture des molécules de liaison étant déclenchée par des bactéries entrantes.

12. Implant chirurgical selon la revendication 10, comprenant en outre une couche intermédiaire formée par des groupes fonctionnels réactifs, comprenant de préférence un groupe -OH, le groupe -OH étant fabriqué par traitement au peroxyde d'hydrogène sous pression et une série de procédés de chauffage, ou un groupe amide (-NH₂), le groupe amide étant fabriqué par implantation d'ions par immersion dans un plasma d'azote, d'oxygène et d'ammoniac, sur la surface de l'alliage de titane traité par plasma, la couche intermédiaire facilitant le couplage de molécules de liaison sur la surface de l'alliage de titane traité par plasma et facilitant l'agrégation de peptides antimicrobiens lors de la formation du revêtement de peptides antimicrobiens à libération contrôlable.

13. Implant chirurgical selon la revendication 12, dans lequel le groupe fonctionnel réactif comporte un groupe -OH, dans lequel la molécule de liaison comporte de l'APTES, dans lequel l'APTES se fixe à une extrémité au groupe -OH sur la surface de titane traité par plasma, dans lequel une autre extrémité de l'APTES ayant un groupe amine primaire se fixe à des lieurs et/ou des peptides antimicrobiens supplémentaires et le groupe amine primaire à l'autre extrémité de l'APTES est transformé en un deuxième groupe fonctionnel par une série de réactions chimiques.
